# FASCICULE DE BREVET EUROPEEN

(11) **EP 3 666 226 B1**
(45) Date de publication et mention de la délivrance du brevet: **28.12.2022**
(21) Numéro de dépôt: 19315146.1
(22) Date de dépôt: 26.11.2019
(51) Int. Cl.: A61F 2/00

(54) **IMPLANT D'AUGMENTATION DU VOLUME DES TESTICULES CHEZ L'HOMME**
IMPLANTAT ZUR VOLUMENVERGRÖSSERUNG DER HODEN BEIM MANN
IMPLANT FOR INCREASING THE VOLUME OF THE TESTICLES IN A MAN

(43) Date de publication de la demande: 17.06.2020
(73) Titulaire: Athmani, Bachir, 75017 Paris (FR); Abecassis, Marc, 75017 Paris P (FR)
(72) Inventeur: Athmani, Bachir, 75017 Paris (FR); Abecassis, Marc, 75017 Paris P (FR)

(56) Documents cités:
- WO-A1-2006/133366
- FR-A1- 3 037 229
- US-A- 5 662 709
- FERNANDO UGARTE Y ROMANO ET AL: "Testicular Augmentation Using Chin Implants", JOURNAL OF SEXUAL MEDICINE, vol. 10, no. 10, 1 octobre 2013 (2013-10-01), pages 2582-2585, XP055269342, GB ISSN: 1743-6095, DOI: 10.1111/j.1743-6109.2012.02963.x

## Description

Le document FR 3 037 229 A1 divulgue une prothèse d'agrandissement testiculaire à visée esthétique en forme de « coque » de forme ovale, enveloppant le testicule et présentant une fente latérale pour l'épididyme.

L'invention concerne un implant selon l'objet de la revendication indépendante 1 mis en place dans la bourse scrotale à travers une courte incision chirurgicale.

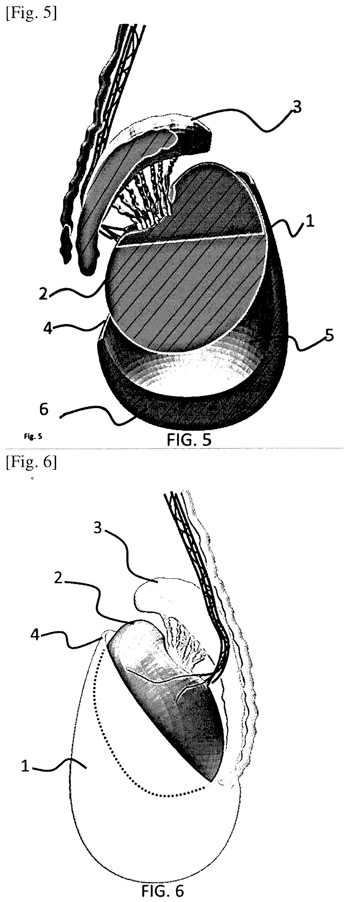

Cet implant couvre une grande partie du testicule (2), en étant en contact direct seulement par les bords périphériques (4) et en laissant à découvert l'épididyme (3) [Fig. 5] et [Fig. 6]

Bon nombre de patients se plaignent de la taille et / ou de la forme des testicules. Cet implant, en améliorant l'aspect esthétique et en augmentant le volume de ces derniers, a le but de pallier aux complexes de ces patients et de pouvoir leur procurer une vie intime sereine, en harmonie avec leur corps.

Cet implant répond à cette demande. Il est souple, flexible, naturel et n'interfère pas dans la fonction du testicule en place.

L'implant a la forme ovale, d'une coquille d'œuf, tronquée de son tiers supéro-externe selon un axe oblique de haut en bas [Fig. 1]. La surface tronquée devient la face d'ouverture frontale par laquelle s'emboitera le testicule [Fig. 2] et [Fig. 3]

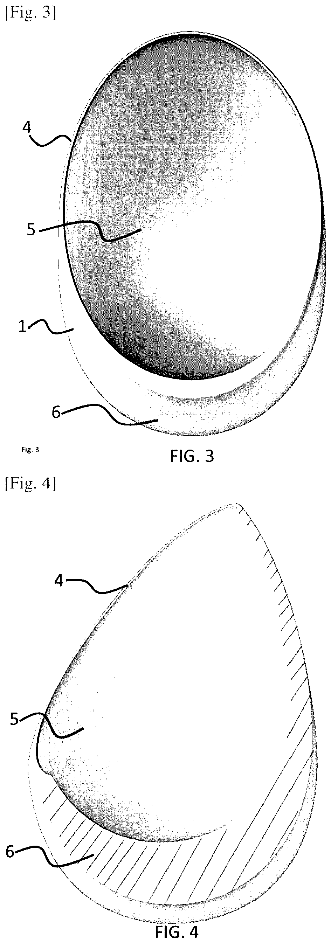

L'implant présente une face externe convexe dans tous les sens et une face interne concave (5) et un bord périphérique (4) profilé dont l'épaisseur augmente vers la base (6). [Fig. 4]

La voie d'abord pour la mise en place de l'implant peut être médiane, dans le raphé scrotal ou latérale dans un pli naturel du scrotum.

L'augmentation testiculaire, selon le cas peut être unilatérale ou bilatérale.

Points particuliers et innovants sont :

1 / La surface de contact entre le testicule et l'implant n'est que partielle et limitée préservant ainsi l'intégrité physique du testicule [Fig. 5]

2/ Le testicule partiellement couvert et non pas encerclé par l'implant, autorise un accès facile en vue de sa palpation et de son exploration [Fig. 6]

3/ la forme évidée de l'implant donne une grande légèreté et une grande souplesse, en comparaison avec d'autres implants existants et d'autre part le renforcement au niveau de la base fournit une plus grande stabilité. [Fig. 4]

4/ la forme en coquille d'œuf donne à cet implant un aspect naturellement esthétique et harmonieux [Fig. 6]

5/ Grace à ces caractéristiques techniques et morphologiques, la pose de l'implant est rendue relativement simple par le simple mouvement de glissement de l'implant à travers l'incision scrotale.

6/ de la même façon, une explantation de l'implant, pour des raisons médicales et/ou personnelles est rendue aisée grâce à un accès facile, puisque le testicule n'est pas entouré complètement, et reste ainsi mobilisable à souhait.

Les dessins annexés illustrent l'invention :

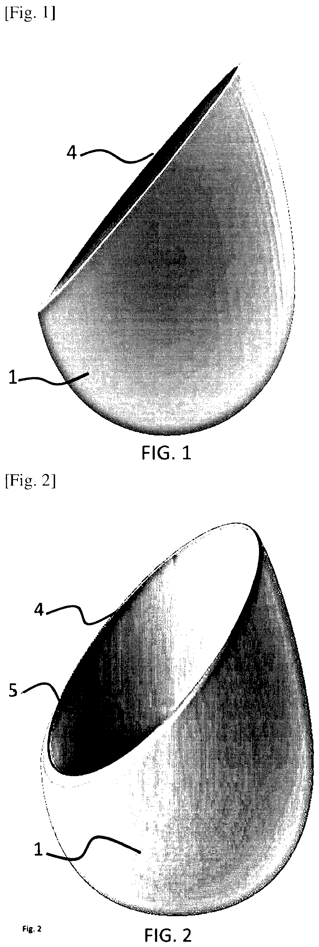

[Fig. 1] représente une vue de profil de l'implant.
[Fig. 2] représente une vue de ¾ de l'implant.
[Fig. 3] représente une vue de face de l'implant
[Fig. 4] représente une coupe sagittale de l'implant
[Fig. 5] représente une coupe sagittale de l'implant en position avec le testicule
[Fig. 6] représente une vue de profile de l'implant en position avec le testicule

## Revendications

1. Implant d'augmentation du volume d'un testicule chez l'homme en silicone ou bien en tout autre matériau biocompatible souple léger et flexible, de forme ovale en coquille d'œuf tronquée de son tiers supéro- externe selon un axe oblique de haut en bas (1), l'implant comportant une face externe convexe dans tous les sens et une face interne concave (5) et le bord périphérique (4) de l'implant étant profilé de sorte que son épaisseur augmente vers la base (6).

2. Implant selon la revendication 1 **caractérisé en ce qu'**il comporte, au niveau de sa surface tronquée, une face d'ouverture frontale configurée pour l'emboitement d'un testicule (2).

3. Implant selon la revendication 1 et la revendication 2 **caractérisé en ce qu'**il est configuré pour couvrir une grande partie du testicule (2), en étant en contact direct seulement par le bord périphérique (4) et en laissant à découvert l'épididyme (3).

## Patentansprüche

1. Implantat aus Silikon oder einem anderen weichen und flexiblen Material in Eiform, das im oberen Drittel entlang einer schräg von oben nach unten verlaufenden Achse abgeschnitten ist (1). Es zeichnet sich durch eine konvexe Außenseite und eine konkave Innenseite (5) und einen äußeren Rand aus (4), der im Profil nach unten hin dicker wird (6).

2. Implantat nach Anspruch 1 **dadurch gekennzeichnet, dass** es an der abgeschnittenen Fläche eine frontale Öffnung aufweist, in die der Hoden (2) eingebettet wird.

3. Implantat nach Anspruch 1 und Anspruch 2 **dadurch gekennzeichnet, dass** es einen Großteil des Hodens bedeckt (2), wobei es damit nur am äußeren Rand (5) in direktem Kontakt kommt (4) und den Nebenhoden (3) frei lässt. |

## Claims

1. Implant in silicone or any other supple, light and flexible biocompatible material, with an oval egg-shaped design, truncated from its superolateral third along an oblique axis from top to bottom. (1), **characterised in that** it comprises a convex outer side in all orientations, a concave inner side (5) and a profiled peripheral edge (4) the thickness of which increases towards the bottom (6).

2. Implant according to claim 1 **characterised in that** it comprises, on its truncated surface, a frontal opening side designed for encasing a testicle (2).

3. Implant according to claim 1 and claim 2 **characterised in that** it covers a large area of the testicle (2), being in direct contact only with the peripheral edge (4) and leaving exposed the epididymis (3).
